**Europäisches Patentamt**

**European Patent Office**

**Office européen des brevets**

(19)

(11) Publication number : **0 484 148 A1**

# (12) EUROPEAN PATENT APPLICATION

(21) Application number : **91310049.1**

(22) Date of filing : **31.10.91**

(51) Int. Cl.⁵ : **A61K 39/40,** C07K 3/26,
// (A61K39/40, 31:65),
(A61K39/40, 31:43),
(A61K39/40, 31:41)

(30) Priority : **01.11.90 JP 296609/90**

(43) Date of publication of application :
**06.05.92 Bulletin 92/19**

(84) Designated Contracting States :
**BE CH DE DK FR GB IT LI NL**

(71) Applicant : **IMMUNO JAPAN INC.**
**4-28-14-701, Ogikubo Suginami-Ku**
**Tokyo 167 (JP)**

(72) Inventor : **Ando, Kunio**
**1877, Shimosakunobe**
**Takatsuku, Sawasaki 213 (JP)**
Inventor : **Nakamura, Tetsuo**
**4-28-14-701, Ogikubo, Suginami-ku**
**Tokyo 167 (JP)**

(74) Representative : **Arthur, Bryan Edward**
**4 Dyers Buildings Holborn**
**London, EC1N 2JT (GB)**

(54) **A method for producing a new medicine for both treating and preventing peptic ulcer diseases and gastritis and thusformulated medicines.**

(57)    A method for producing a new medicine which is useful for both treating and/or preventing peptic ulcer diseases and gastritis (through the eradication of *Helicobacter pylori* colonizing the upper gastrointestinal tract) by anti-*Helicobacter pylori* antibodies which are extracted either from blood or milk of domestic animals, and the methods for preparing the medicines containing anti-*Helicobacter pylori* antibodies of either milk or blood origins as an active ingredient.

EP 0 484 148 A1

## Background to the Invention

The present invention relates to a method of treating and/or preventing peptic ulcer diseases and gastritis in warm bloodied mammals (including humans) through the eradication of *Helicobacter pylori* (colonizing the upper gastrointestinal tract) by use of anti-*Helicobacter pylori* antibodies which are typically extracted either from blood or milk of domestic animals. The present invention also relates to methods for preparing medicines containing anti-*Helicobacter pylori* antibodies, typically of either milk or blood origin, as an active ingredient. In a further aspect, the present invention relates to pharmaceutical compositions for treating and/or preventing peptic ulcer diseases and gastritis in mammals.

The present invention relates to the production of a new medicine used for treating and/or preventing diseases of the upper gastrointestinal tract (such as peptic ulcers and gastritis), and formulated medicines which are mixed with or without antimicrobial agents effective against *H. pylori.* The active ingredient of the new medicine of the present invention is anti-*Helicobacter pylori* polyclonal antibodies found in milk (especially in colostrum) and blood of large domestic animals, whether or not they have previously been immunized with *H. pylori.* Anti-*H. pylori* antibodies are rather ubiquitously present both in blood and milk of most domestic animals and human beings, although the mechanism of infection and colonization by *H. pylori* in the human upper gastrointestinal tract has not been clarified yet. The present invention also relates to formulated medicines containing the anti-*H. pylori* antibodies produced by the present method.

The diseases most frequently encountered in the upper gastrointestinal tract are gastritis and peptic ulcers. At present these syndromes are successfully treated with miscellaneous drugs, among which the first choice are class $H_2$ blockers which specifically bind to histamine #2 receptor ($H_2$ receptor) in the gastric wall, thus inhibiting gastric acid secretion in the stomach by blocking the binding of histamine to its receptor. Gastric histamine is released in response to the stimuli of food intake and its binding to $H_2$ receptor triggers gastric acid secretion into the stomach. Other remedies include proton pump inhibitors which also diminish gastric acid secretion by inhibiting production of acid in the gastric wall, and so-called cytoprotective agents which protect gastric mucosa from corrosive attack by gastric juice. The introduction of $H_2$ blockers into gastroenterology was an epoch-making event for both treatment and prevention of upper gastrointestinal syndromes since 90% of peptic ulcers are cured due to these drugs without any surgical operation. However, it was gradually revealed that the $H_2$ blockers are not miraculous but possess a number of serious defects. The most serious $H_2$ blocker defect is the high recurrence rate after healing; for example, after an ulcer is completely healed (observed endoscopically), it recurs within 1 year at unusually high frequencies of 70-90% unless medication with $H_2$ blocker persists. Therefore, the patients have to continue to take $H_2$ blockers after the symptoms have disappeared. This is a heavy burden both for the individual and for society because the daily drug intake is mentally troublesome as well as an economic burden for those who recovered from the illness. In addition, the cost for preventing the recurrence of peptic ulcer is considerably high for the social security system and the problem is increasing. Therefore, a need has existed for a therapy which can lead to complete cure of upper gastrointestinal disorders without requiring the medicament after the symptoms have disappeared.

Recently it was discovered that upper gastrointestinal diseases (such as type B active chronic gastritis, peptic ulcers and gastric cancer) are closely associated with unique bacteria colonizing the stomach, and it has become gradually evident that the colonizing bacteria, *H. pylori*, contribute to the pathogenesis of the upper gastrointestinal syndrome.

Since the late 19th century, some pathologists have observed the presence of spiral bacteria on the surface of gastric mucosa and have suggested the bacteria as the causative agents in the etiology of upper gastrointestinal disorders. For example, Dominges found spirochete-like organisms on the surface of gastric mucosa obtained from autopsy specimens of gastric ulcer patients (Proc. Soc. Exp. Biol. Med., 38:536-8, 1938). Freedberger and Baroon also observed similar organisms on the gastric mucosa obtained from subjects who suffered from upper gastrointestinal disorders (Am. J. Digestive Dis., 7:443-5, 1940). The presence of this organism was unusually high in the patients since 13 out of 35 subjects retained this organisms in their stomach. In 1975, Steer reported that numerous bacteria were seen sandwiched between the mucous layer and epithelia of gastric mucosa, and in polymorphonuclear leucocyte infiltrates in the infected loci.

However, the dogma prevailing at that time was that the stomach would be free from bacterial colonization due to its strong acidity. Bacteria, if observed on the surface of gastric epithelia, were regarded either as an artifact, or only passengers, but not inherent inhabitants in the stomach. The consensus in gastroenterology was that the major risk factors for upper gastrointestinal diseases were deemed to be excessive intake of alcohol, salt and foods as well as mental stress, but not bacterial infection.

In 1983, Warren and Marshall reported that bent and spiral bacilli were colonizing on the gastric epithelia of biopsy specimens endoscopically taken from patients with chronic gastritis. These bacteria were never seen in the biopsy specimens taken from normal healthy volunteers. Therefore, they claimed that the gastric cavity is not bacteria-free but allows bacterial growth and colonization despite its strong acidity, and further studies would be needed to confirm whether or not the bacteria are a causative agent of upper gastrointestinal diseases. Marshall succeeded in isolating and culturing the bacteria under microaerobic conditions ($CO_2:O_2=95:5$) using the same culture medium as that for isolating *Campylobacter* species from the feces of diarrheal patients. The morphological aspects of these unusual new bacteria have been clarified through electron microscopy and negatively stained specimens. Ultrastructural studies show this organism, *Helicobacter pylori*, as spiral, unipolar multiflagellate cells with bluntly rounded ends, measuring 0.5-1.0 $\mu$m in width and 2.5-4.0 $\mu$m in length.

Although correlation between *H.pylori* and gastric disorders such as peptic ulcers and gastritis had not conclusively been elucidated yet, much attention was focused on these unusual bacteria and the research has advanced very quickly. Until now much research and discussion by gastroenterologists has led to the consensus that the colonization by this organism of the upper gastrointestinal tract is closely correlated to duodenal ulcer and active chronic gastritis type B, and possibly gastric ulcer and gastric cancer. For example, at the international conference of gastroenterology held at Sydney, Australia in 1990, the workshop on *H. pylori* concluded that infection by and propagation of *H. pylori* plays a primary role in the pathogenesis of duodenal ulcer and its eradication from the stomach is crucially important for healing the disease. In addition, another working group at the conference stated that colonization by *H. pylori* also closely correlates with type B active chronic gastritis in human beings. Moreover, it was gradually revealed that occurrence of gastric cancer is accelerated by the colonization of the stomach by *H. pylori*. Therefore, it is apparent that there is highly significant correlation between *H. pylori*'s colonization of the stomach and the diseases of the upper gastrointestinal tract.

The eradication of *H. pylori* from the stomach and the persistence of the colonization-free condition are conclusively important for treating these diseases since recurrence of the disease occurs simultaneously with recolonization by *H. pylori*. Therefore, a drug which is able to eradicate colonizing *H. pylori* from the stomach possibly becomes the most important treatment of duodenal ulcer and active chronic gastritis, and also for prevention of upper gastrointestinal diseases including gastric cancer.

## SUMMARY OF THE INVENTION

One object of the present invention was to find a new efficient medicine for treating and/or preventing the diseases of the upper gastrointestinal tract in warm blooded mammals, including humans, caused by colonization of the mammalian stomach by *H. pylori*. This and other objects of the present invention can be attained by a therapeutic method involving oral administration of anti-*H. pylori* immunoglobulins. Typically these are extracted and purified either from milk (especially colostrum) or blood.

The methods for extraction and purification of anti-*H. pylori* immunoglobulins comprise either selective precipitation (e.g., with ammonium sulfate), or ion-exchange, or gel-filtration and affinity column chromatography, or combinations of these. The anti-*H. pylori* immunoglobulins specifically bind to *H. pylori*'s antigenic determinants (epitopes), thus killing the bacteria in cooperation with the host defense system of infected mammals. The immunoglobulins being able to bind to the epitopes of *H. pylori* are found in the sera and milk of human beings and other mammals such as domestic animals (e.g., cows, goats, sheep and pigs) and can be isolated in relatively pure form. However, large animals immunized with whole cells of *H. pylori* (with or without adjuvants) are more of a desirable source to obtain immunoglobulins with much higher binding activity against *H. pylori*.

Another object of the present invention was to formulate the effective medicines (i.e., pharmaceutical preparations) containing the antibodies prepared by the method shown in this invention.

## DETAILED DESCRIPTION OF THE INVENTION

*Helicobacter pylori* are highly sensitive to several varieties of antimicrobial agents, including beta-lactam and tetracycline antibiotics. Therefore, some years ago it was considered that persistent administration of these antibiotics was the safest way to eradicate the bacterial colonies from the stomach. Unexpectedly, antibiotic medication alone had virtually no effect on colonization by *H. pylori* and consequently failed to cure the patients. The colonies are covered with a thick hydrophobic mucus layer and are protected from gastric acid so that sufficient amounts of antibiotics can not reach the colonization loci. In addition, oral antibiotics quickly pass through the stomach so that the colonies are exposed to the action of antibiotics for a relatively short time. These are the major reasons why antibiotics alone are ineffective on the eradication of the colonies. The potent chemotherapies frequently used against *H. pylori* are beta-lactams, amoxicilin and ampicillin, oxytetracycline, macrolide erythromycin, and the synthetic antimicrobial agent tinidazole. These agents can inhibit the growth of *H. pylori* at very low concentrations *in vitro*, but monotherapy with these agents failed to eradicate *H. pylori* from the stomach. Bayerdoerffer and Ottenhann (Scand. J. Gastroenterol., 23 (suppl. 142): 93-100, 1988) reviewed the efficacy of antimicrobial agents against *H. pylori* and compared the eradicating activity between antibiotic alone, tinidazole, bismuth subcitrate, and the combination of these agents; amoxicillin alone for 2 weeks showed an eradication rate of 26% 12 month after the medication which is rather ineffective on eradication, especially since in another report the eradication rate was 0% 2 weeks after the amoxicillin administration. The combined therapy of antimicrobials with colloidal bismuth subcitrate yielded a high eradication rate even after 12 months; in the case of combination therapy of bismuth subcitrate with erythromycin, 4 weeks of medication resulted in a 65% eradication rate, with amoxicillin 47-50%, with tinidazole 75%.

The minimum inhibitory concentration of bismuth subcitrate is more than 64 μg/ml, which is unattainably high in the stomach. Therefore, another explanation is necessary for its mode of action. *Helicobacter pylori* can readily acquire resistance to the various antimicrobial chemotherapies, and bismuth subcitrate can efficiently retard the acquisition of resistance against the antimicrobial chemotherapies. Moreover, the bismuth salt possesses an epidermal growth factor-like activity, thus accelerating ulcer healing. The third and fourth mechanisms of bismuth subcitrate are that it increases the production of carbonic salt *in vivo* and potentiates antibiotic activity by binding on the cells of *H. pylori*.

Therefore, the combination of bismuth subcitrate with antimicrobial chemotherapies hopefully eradicates *H. pylori* colonizing the stomach, and the research activity regarding this eradication therapy is increasing. Because the eradication therapy can heal damaged gastric walls by a relatively short time of 1-4 weeks, use of life-long medicament like $H_2$ blockers will be unnecessary for the major portion of recovered subjects. If this is true, it will remove the burden from individuals and the medical system. This is the strong point of eradication therapy in comparison with the $H_2$ blockers which require life-long administration. The weak point of the eradication therapy currently in use is its frequent side-effects, although these are not severe; for example, during 4 weeks of medication, 25% of patients suffered from nausea, 11% suffered from diarrhea, and 5% suffered from vertigo. Another weak point is its neurotoxicity; it is well known that bismuth is a neurotoxin, and accumulation leads to irreversible severe damage in the central nervous system.

After studying upper gastrointestinal diseases for many years, a new efficient medicine for treatment and/or prevention of the diseases cited above has now been found by Applicants. Thus, it has been found that anti-*H. pylori* immunoglobulins (which can aggregate the cells of *H. pylori*) are present in the blood and milk of most mammals tested, and oral administration of isolated anti-*H. pylori* immunoglobulins (with or without antimicrobial chemotherapies) efficiently eradicates *H. pylori in vivo* and significantly stimulates the healing processes of upper gastrointestinal diseases without any side effects. Moreover, oral administration efficiently prevents recurrence of peptic ulcer at high frequency. The strongest point of this medicament is the low recurrence rate due to the eradication of colonizing *H. pylori* as compared with other medicaments such as $H_2$ blockers and cytoprotective agents.

This medicament, or therapeutic pharmaceutical composition, is suitable for treatment and/or prevention of upper gastrointestinal diseases and is characterized by the features summarized as follows:

1. The oral medicine can contain anti-*H. pylori* polyclonal immunoglobulins, which can aggregate the cells of *H. pylori*, as active ingredient and is used for the treatment and/or prevention of upper gastrointestinal diseases.
2. The oral medicine can contain anti-*H. pylori* polyclonal immunoglobulins (isolated from milk, especially colostrum) for the treatment and/or prevention of upper gastrointestinal diseases.
3. The oral medicine can contain anti-*H. pylori* polyclonal immunoglobulins (isolated from the blood of mammals) for the treatment and/or prevention of upper gastrointestinal diseases.
4. The oral medicine can contain anti-*H. pylori* polyclonal immunoglobulins (isolated from mammalian blood and milk) combined with antimicrobial chemotherapies effective against *H. pylori* as active ingredients for the treatment and/or prevention of upper gastrointestinal diseases.

In addition to the above, the present invention features a process for manufacturing anti-*H. pylori* immunoglobulins (which can aggregate the cells of *H. pylori*) from blood of mammals which have previously been immunized with *H. pylori*. In one embodiment, immunoglobulins from whey (obtained from milk) and from blood are obtained by using an ultrafiltration membrane which is impermeable to protein molecules with molecular weights larger than 100k daltons.

According to another embodiment of the invention, the process for producing anti-*H. pylori* immunoglobulins begins with whey and uses an ultrafiltration membrane which is impermeable to protein molecules with molecular weights larger than 300k daltons.

Still further, the process of the invention for producing anti-*H. pylori* immunoglobulins (from blood and whey) can be carried out using affinity column chromatography where the column is packed with beads embedded with cells of *H. pylori* as adsorbent.

In carrying out the present invention, *H. pylori* strain NCTC 11637, an international standard strain, and clinical isolate #153 were used. *Helicobacter pylori* was not found in the stomachs of cows, goats, sheep, swine or horses, but it is believed that these animal species probably have colonizing microorganisms with antigenic determinants similar to those of *H. pylori* because they have immunoglobulins which cross-react to the strains of *H. pylori* found in humans.

It is not clear how many antigenic determinants are present on the surface of *H. pylori*. The present invention confirmed that 50/52 fresh clinical isolates of *H. pylori* from patients with gastritis react with bovine milk immunoglobulin (which was prepared from colostral whey through ultrafiltration with a cut-off value of 100k daltons) as shown in Table 1.

TABLE 1.   Agglutination titers of bovine colostral immunoglobulin preparations against 50/52 clinical isolates of *H. pylori*.

| minimal agglutinable concentration (mg/ml) | lot numbers of immunoglobulins | | | | | |
|---|---|---|---|---|---|---|
| | 1 | 2 | 3 | 4 | 5 | 6 |
| 0.32 | 0 | 1 | 0 | 2 | 1 | 0 |
| 0.64 | 3 | 1 | 0 | 0 | 1 | 0 |
| 1.28 | 7 | 4 | 2 | 6 | 12 | 9 |
| 2.56 | 30 | 31 | 34 | 28 | 36 | 27 |
| 5.12 | 11 | 8 | 11 | 13 | 0 | 13 |
| 20.48 | 0 | 3 | 0 | 1 | 0 | 1 |

Table 1 indicates each lot of purified bovine immunoglobulin agglutinates the cells at very high dilution rates (with an average titer of 2.56 µg/ml) and the difference between each lot was small. This fact indicates that the antibodies derived from animal species other than human reacts well to strains of *H. pylori* found in humans. The minimum concentration of anti-*H. pylori* antibodies required for agglutination of the cells of *H. pylori* is so small that drinking 200 ml of cow's milk is sufficient for agglutinating *H. pylori* unless the immunoglobulin is denatured. In cow's milk immunoglobulins account for 10% of total whey proteins at an approximate concentration of 600-700 µg/ml. However, the immunoglobulins are highly sensitive to heat, nearly 100% are inactivated by a heat sterilization process.

The fact that cows possess high titers of anti-*H. pylori* antibodies in blood and milk indicate that they have similar bacteria with common antigenic determinants to *H. pylori* colonizing somewhere in their huge volume of stomach. Cow's milk with high titers of anti-*H. pylori* antibodies protects their neonates from lethal infection by this organism; their neonates have no immunoglobulins in blood at birth and are highly susceptible to lethal infection by microorganisms and viruses. It is not clear why such kinds of antibodies are present both in blood and milk of cows. Supposedly, infection by the organism with the common antigenic determinants to *H. pylori* may be fatal for neonates because the organism causes severe damages in the upper gastrointestinal tract resulting in injury to the digestive process and absorption of nutrients.

Bovine colostral immunoglobulin used in the test was prepared as follows: the colostrum was collected postnatally for 4 days, was defatted by centrifugation, rennet was added to the aqueous layer at pH 4.6 in the presence of Ca ion, was filtered with cheese cloth, and the resulting whey was passed through an ultrafiltration membrane which is impermeable to proteins with molecular weights larger than 100k daltons. The retentate was lyophilized yielding immunoglobulin rich powder (approximate 75% purity). This process lasts for a considerable time so the retentate is apt to be contaminated with microorganisms. To avoid microbial contamination, it is desirable to pass the whey through a clarifying filter with pore size less than 0.2 µm to remove contaminating microorganisms before ultrafiltration.

The ultrafiltration module is equipped with an ultrafilter impermeable to protein molecules with molecular weights larger than 100k daltons, so it is very convenient to separate the immunoglobulins not only from low molecular weight compounds but also from milk proteins having molecular weights less than 100k daltons because the immunoglobulin with the lowest molecular weight is immunoglobulin G (140k daltons). By this procedure, alpha-lactoalbumin, beta-lactoglobulin and serum albumin are significantly removed from the retentate and immunoglobulin content is much improved.

Bovine milk immunoglobulins contain immunoglobulin $G_1$ ($IgG_1$), $G_2$ ($IgG_2$), A (IgA) and M (IgM) at an approximate ratio of 85-2:3:10; their precise molecular weights are 153,000-163,000 for $IgG_1$, approximately 146,000 for $IgG_2$, 385,000-417,00 for sIgA, and 960,000-1,000,000 for IgM; and the sedimentation coefficients are 6.3-7.0, 6.5-7.1, 10.8-11.1 and 18.2-19.8, respectively, The isoelectric points are 5.5 for $IgG_1$ and 7.5-8.3

for $IgG_2$.

Ammonium sulfate precipitation is the simplest way of purifying immunoglobulins from whey and serum. However, the yield calculated from the theoretical base is usually as low as 50%, and large amounts of ammonium sulfate have to be discarded. This is apt to cause environmental pollution. Therefore, the cost/performance ratio of the ultrafiltration process is much superior to that of ammonium sulfate precipitation for isolation and purification of immunoglobulins. Highly purified immunoglobulins can be obtained as colorless powder with slight milk flavor by using either method. Spray-drying and lyophilization are suitable for obtaining the immunoglobulins in powdered form.

The purified immunoglobulin thus obtained can agglutinate the cells of *H. pylori* at a dilution rate of $10^6$ to $10^7$. Therefore, oral administration of 0.5-1.0 g/day is sufficient enough to treat and prevent gastritis and duodenal ulcer in patients.

Furthermore, affinity chromatography is extremely useful for separating anti-*H. pylori* antibodies from other antibodies. In this case, the antigen is the dead cells of *H. pylori* (the live cells of which are either treated with 5% formaldehyde or irradiated with ultraviolet light, and the dead cells are collected, dried and embedded into the appropriate carriers (such as the gels of polyacrylamide and alginic acid) making minute beads). The solution containing anti-*H. pylori* antibodies passes through the column packed with these beads and the antibodies adsorbed are eluted with saline or sodium thiocyanide resulting in antigen-specific purification of anti-*H. pylori* antibodies where the specific activity increases more than 100-1000 times.

Another way for producing anti-*H. pylori* antibodies with extremely high titer is to immunize animals with *H. pylori* (with or without adjuvants). This method is useful for commercial production of antibodies with high specific activity against *H. pylori*. For example, the anti-*H. pylori* titer in milk is elevated 50-100 times by immunizing cows with the dried cells of *H. pylori* (2 mg mixed with Freund's complete adjuvant each time) for two times (initiating 6 weeks before parturition).

The anti-*H. pylori* antibodies with high specific activity can successfully treat patients with gastritis and duodenal ulcer at daily doses of 0.01-0.1 g/day within 4 weeks and shows no side effect.

Thus, anti-*H. pylori* immunoglobulins are very useful for treating and/or preventing upper gastrointestinal diseases, but the weak point is that the antibodies alone possess no killing action against the bacteria, although it inhibits their growth. The combined therapy of the antibodies with antimicrobial chemotherapies including antibiotics can overcome this difficulty. For example, 37 *H. pylori* positive gastritis and duodenal ulcer patients, who had endoscopically proven gastric colonization by *H. pylori*, including the recurrence cases, were randomly allocated into 3 groups. The first group (n=13) was orally administered 1 g/day of anti-*H. pylori* antibodies for 14 days (specific activity; maximal dilution rate of $10^7$. Three months after the medicament, the eradication rate was 62% (8/13), 2 patients out of 13 recurred, and *H. pylori* was detected from biopsy specimens of the recurred patients indicating that re-colonization occurred simultaneous with recurrence. The second group (n=12) received the same dosage of antibodies (1 g/day) together with tetracycline (250 mg/day) and metronidazole (400 mg/day) for 14 days, and the eradication rate was 100% (P<0.01), even 3 months later no recurrence occurred. The third group (n=12) orally received the same dosage of antibodies (1 g/day) for 3 months. In this group the symptoms disappeared 4 weeks later and no recurrence occurred 3 months after the medicament showing that long term administration of anti-*H. pylori* antibodies is clearly curative but an administration period shorter than 1 month is insufficient for complete eradication of *H. pylori*. It is worth noting that the latter 2 methods bring about complete eradication of *H. pylori* colonizing the gastric wall.

The immunoglobulin powder is rather hydrophobic, although its solubility is considerably high in saline. When added to water or saline, it floats on the surface and it takes a long time to dissolve completely. The loci where *H. pylori* is colonized are under hydrophobic mucus layers in the stomach. Hence, it is anticipated that the dissolving velocity of immunoglobulin molecules is crucially important for access to the loci of colonization, the staying time of the antibodies is relatively short in the stomach. The dissolving velocity is accelerated by micronizing the immunoglobulin particles and by mixing with hydrophilic vehicles such as monosaccharides or disaccharides under slightly moistened condition. By using these methods the resulting immunoglobulin preparations show a much faster dissolving velocity than the original powder.

To prepare immunoglobulin particles with diameters less than 40 μm, a specialized spray-drying method is applied. A diluted immunoglobulin solution (with concentration less than 5%) is rapidly sprayed through a nozzle having a very small diameter. An alternative method to prepare particles with diameters less than 40 μm is to pulverize the lyophilized powder with a micronizing machine which utilizes physical energy to crush the large particles.

When administering the immunoglobulins to mammals orally, any formulated form may be used; e.g., tablets, granules, powder, syrup and so on. The immunoglobulins can be utilized when mixed with foods, e.g., supplemented with milk, yogurt, skim milk powder, lactic acid bacteria fermented milk, chocolates, table sweets, and powdered beverages. The most important factor for producing the immunoglobulin-containing forms is to avoid protein denaturation during processing by not exceeding a temperature of about 60°C.

The pharmaceutical composition of this invention may contain the active compounds together with a solid or liquid pharmaceutically acceptable nontoxic carrier. Such pharmaceutical carriers can be sterile liquids, such as water. Saline solution and aqueous dextrose and glycerol solutions can also be employed as liquid carriers. Suitable pharmaceutical excipients include starch, glucose, lactose, sucrose, gelatin, malt, rice flour, chalk, silica gel, magnesium carbonate, magnesium stearate, talc, sodium chloride, dried skim milk, glycerol, propylene glycol, water, ethanol, and the like. Monosaccharides, such as glucose, galactose, fructose, xylose, arabinose, sorbitol and others, and disaccharides, such as sucrose, lactose, maltose, isomaltose and others, are very suitable carriers for endowing hydrophilic property to the immunoglobulin particles when preparing the tablets, granules, and powdered form preparations. These compositions can take the form of solutions, suspensions, tablets, pills, capsules, powders, sustained release formulations and the like. Suitable pharmaceutical carriers are described in "Remington's Pharmaceutical Science" by E.W. Martin. Such compositions will contain an effective therapeutic amount of the active compound together with a suitable amount of carrier so as to provide the form for proper administration to the host. The only route for administering anti-*H. pylori* immunoglobulins is the oral one, and other modes can not be employed. Antimicrobial chemotherapies including antibiotics can be mixed with anti-*H. pylori* antibodies; pharmaceutical preparations in any oral form can be successfully prepared using the above mentioned methods.

**Examples**

Example 1.:

The specific activity of anti-*H. pylori* immunoglobulins was titrated using the hemagglutinin method of sheep red blood cells against the formaldehyde-treated cells of *H. pylori* No. 153 (which was isolated and identified by the present inventors from a biopsy specimen of a patient with gastritis upon endoscopic examination). The titers of milk and colostra are shown in Table 2 (hemagglutinin titer of milk and colostrum from mammals).

Table 2.  Specific activity of anti-*H. pylori* antibodies in mammals

| animal species | | titer dilution rate | animal species | | titer dilution rate |
|---|---|---|---|---|---|
| cow* | colostrum<br>milk | $2-4 \times 10^5$<br>$5-7 \times 10^2$ | human | colostrum<br>milk | $0-6 \times 10^4$<br>$0-2 \times 10^2$ |
| goat* | colostrum<br>milk | $1-2 \times 10^5$<br>$5 \times 10^2$ | dog | colostrum<br>milk | not tested<br>$< 10^2$ |
| sheep* | colostrum<br>milk | $1-2 \times 10^5$<br>$5 \times 10^2$ | cat | colostrum<br>milk | not tested<br>$< 10^2$ |
| horse | colostrum<br>milk | $< 1 \times 10^2$<br>$< 1 \times 10^2$ | swine | colostrum<br>milk | $0-1 \times 10^5$<br>$0-1 \times 10^2$ |

*)  Immunized with an admixture of dead *H. pylori* NCTC 11362 cells and Freund's complete adjuvant.

As shown in this example, mammalian milk, especially cow's milk, can be successfully utilized as sources for anti-*H. pylori* antibodies. Colostrum is much better than milk since the immunoglobulin content of the former is several times higher than the latter.

Mammalian blood is also useful as sources for anti-*H. pylori* antibodies. In this case, the first step for purification is selective precipitation either by alcohol or ammonium sulfate.

Example 2:

Colostrum (100 liters) collected from cows within 48 hours after parturition was diluted to 200 liters by the addition of water, and the cream was separated with the use of a cream separator (187 liters of the defatted colostrum was thus obtained), and the pH of the defatted colostrum was adjusted to 4.6 by adding 10% lactic acid solution. Then rennet (0.002%) and calcium chloride (0.02%) was added to the colostrum and the mixture was kept standing at room temperature for 2 hours to coagulate casein curd. The curd was removed by filtration through cheese cloth and the resulting whey fraction was passed through a filter with pore size of 0.2 μm to remove contaminating microorganisms. The filtrate was concentrated by reverse osmosis to 10 liters and the concentrate was diluted with water to 100 liters. The diluted whey was passed through an ultrafiltration module (equipped with an ultrafiltration membrane impermeable to proteins with molecular weights larger than 100k daltons) and the volume was reduced to 10 liters. Then the retentate was diluted to 100 liters with water and ultrafiltration was repeated once. The final volume of the retentate was 10 liters containing 20% protein;, lyophilization yielded 1.98 kg of immunoglobulin with purity of 83.1% (analysis: protein 98%, lactose less than 1%, ash less than 1%, and fat less than 0.5%).

Example 3:

*Helicobacter pylori* NCTC 11637 was cultured for 3 days at 37°C on the surface of brain-heart infusion agar (Difco) and the cells were collected and suspended in physiological saline. The cell suspension was irradiated for 30 min at a distance of 30 cm with 60 W ultraviolet light for sterilization. Then the cells were collected by centrifugation, washed once with saline, and lyophilized. The dried cells (0.2 g) were suspended in 50 ml of sodium alginate solution and the suspension was added dropwise to 1000 ml of 10% calcium chloride solution. The resulted minute beads were collected and washed thoroughly with water and packed in a glass column. The colostral whey (50 ml, protein content 3%) prepared according to the method described in example 2 was passed through the affinity column. The column was washed with water and eluted with 3M sodium thiocyanide solution. The fractions containing immunoglobulin were collected and the combined eluate was adjusted to a pH 7.0-7.4. Then the impurities were removed by ultrafiltration and the milk protein containing highly enriched anti-*H. pylori* antibodies was obtained in powdered form by lyophilizing the retentate. Analysis: protein >98%, lactose less than 0.5%, ash less than 0.5%, and fat less than 0.5%. The specific activity increased 10-100 times higher than the starting antibody fraction. The recovery rate was 50-70% calculated on the basis of the theoretical value.

Example 4:

A saturated solution (10 liters) of ammonium sulfate was added dropwise to bovine serum (10 liters) at 10°C while gently stirring and the mixture was kept for 3 hours. The clear supernatant was removed by siphoning and the remaining portion containing the precipitate was centrifuged for 20 min at 5,000 x g. The precipitate was resuspended in ammonium sulfate solution (1 liter) with 50% saturation and again centrifuged at 5,000 x g for 20 min. This procedure was repeated twice and the washed precipitate was dissolved in water (2 liters) and pH was adjusted to 7.4. Then the antibody solution was concentrated to 1/5 volume through an ultrafiltration module equipped with a 100k daltons membrane and the retentate (0.2 liter) was diluted by the addition of water. The ultrafiltration was repeated twice and the final retentate was lyophilized to yield bovine immunoglobulin (38 g) as colorless powder. This powder can agglutinate a cell suspension of *H. pylori* at a maximal dilution rate of $1 \times 10^5$.

Example 5:

The bovine colostral immunoglobulin powder (1 kg, purity 65%) prepared by the method described in example 1 was mixed thoroughly with micronized powder of glucose (2 kg); and distilled water (150 ml) was gradually sprayed on the mixture while stirring. After finishing the spraying, the mixture was granulated using a granule-making machine equipped with 2mm net (16-18 mesh). The moistened granules thus prepared were dried at 70°C for 30 min. After drying, the granules (1.7 g) were put into an aluminum pouch and sealed.

Eighteen patients with active chronic gastritis type B, in which colonization by *H. pylori* was confirmed by culturing biopsy specimens and immunological test, were randomly allocated equally into 2 groups. One group (n=9) orally received 2 pouches per day (1 pouch after breakfast and supper each) for 4 weeks, and another group (n=9) also took the same amount of placebo made of lactose. Four weeks after the administration, *H. pylori* was detected no more in the the administration, *H. pylori* was detected no more in the antibody administered group, while one out of 9 patients became negative in the biopsy specimens in the placebo group. The clinical symptoms (sour stomach, dyspepsia and indigestion) were also much improved in the antibody group, whereas the symptoms were not so improved in the placebo group. See Table 3.

Table 3. Efficacy of anti-*H. pylori* antibodies against active chronic gastritis

|  | antibody group | placebo group |
|---|---|---|
| much improved | 8 | 0 |
| improved | 1 | 1 |
| slightly improved | 0 | 1 |
| no change | 0 | 6 |
| worsen | 0 | 1 |

Example 6:

The antibody prepared by the method described in example 1 was micronized using a micronizing machine and the micronized powder (10 kg) was mixed with amoxicillin (4 kg) and lactose (26 kg). The mixture was sprayed with distilled water (1.6 liters) while stirring and granulated as described in example 5. The granules (1.5 g) were packed into an aluminum pouch.

A total of 21 patients with duodenal ulcer, including recurrence cases, were randomly allocated into 2 groups. The first group (n=11, male 8 and female 3) orally took the granules four times a day for 4 weeks and the second group (n=10, male 8 and female 2) orally took one cimetidine tablet (200 mg) after meals and before sleeping for 4 weeks. All of these patients were positive for *H. pylori* before the treatment (biopsy specimens taken under endoscopic examination). Efficacy was assessed by observing the eradication of *H. pylori* from the stomach, and disappearance of clinical symptoms. After 4 week all patients in the combined therapy group became negative for *H. pylori* while 4 out of 10 became negative in the cimetidine group. The cimetidine treatment much improved the clinical symptoms. Improvement similar to that in the cimetidine group was observed in the combined therapy group. See Table 4.

Table 4. Combined treatment against duodenal ulcer

|  | antibody group | cimetidin group |
|---|---|---|
| much improved | 11 | 7 |
| improved | 0 | 1 |
| slightly improved | 0 | 2 |
| no change | 0 | 0 |
| worsen | 0 | 0 |

Example 7:

The antibody (1 kg) prepared by the method shown in example 2 was mixed with lactose (2.5 kg) and tetracycline (0.5 kg) and the mixture was gradually sprayed with water (0.4 liter) while stirring, and granulated. The granules (2 g) were packed in a aluminum pouch to protect from moisture. One pouch was administered twice daily for 2 weeks to 5 patients with duodenal ulcer; *H. pylori* was detected in all biopsy specimens taken immediately before the medicament. After 2 weeks, every biopsy specimen was negative for *H. pylori*, indicating that the combined therapy eradicated *H. pylori* from the stomach. All these patients did not have a recurrence of duodenal ulcer for at least 12 months.

Example 8:

Fresh cheese whey (100 liters) from Cheddar cheese manufacturing was filtered through filter paper and the filtrate was concentrated with reverse osmosis. The concentrate was treated with an ultrafilter (20k daltons) until the volume of retentate was reduced to 1/5. Then the retentate was diluted with water to 50 liters and again treated with ultrafilter (300k daltons) until the volume was reduced to 5 liters. The dilution and ultrafiltration of the retentate were repeated twice to remove alpha-lactoalbumin, beta-lactoglobulin and serum albumin, and the final retentate was lyophilized yielding antibody fraction (57 g). The purity of this fraction was 85% as immunoglobulin $G_1$, and-consisted of IgG 86%, IgM 12% and secretory IgA 2%.

Further variations and modifications of the invention will become apparent to those skilled in the art from the foregoing and are intended to be encompassed by the claims appended hereto.

Japanese Patent Application No. 296609/90, filed on November 1, 1990, is relied on and incorporated by reference.

**Claims**

1.   A method of treating and/or preventing upper gastrointestinal diseases in mammals, said method comprising orally administering to a patient in need thereof an effective amount of a pharmaceutical composition comprising anti-*Helicobacter pylori* polyclonal immunoglobulins and a pharmaceutical acceptable carrier, said effective amount being effective to treat and/or prevent upper gastrointestinal diseases in mammals.

2. The method according to claim 1, wherein 0.01 to 1 gram per day of said anti-*Helicobacter pylori* polyclonal immunoglobulins are orally administered.

3. The method according to claim 1 or 2, wherein said pharmaceutical composition further comprises at least one member selected from beta-lactam and tetracycline antibiotics.

4. The method according to claim 3, wherein said antibiotic is selected from tetracycline, amoxicillin and metronidazole.

5. The method according to claim 4, wherein 250 mg/day of said tetracycline, 400 mg/day of said metronidazole, and 1 g/day of said anti-*Helicobacter pylori* polyclonal immunoglobulins are orally administered.

6. The method according to claim 4, wherein 500 mg/day of said tetracycline and 1 g/day of said anti-*H. pylori* polyclonal immunoglobulins are orally administered.

7. The method according to claim 4, wherein 400 mg/day of said amoxicillin and 1 g/day of said anti-*Helicobacter pylori* polyclonal immunoglobulins are orally administered.

8. A pharmaceutical composition for treating and/or preventing upper gastrointestinal diseases in mammals and humans, said pharmaceutical composition comprising anti-*Helicobacter pylori* polyclonal immunoglobulins and a pharmaceutically acceptable carrier.

9. The pharmaceutical composition according to claim 8, further comprising at least one member selected from beta-lactam and tetracycline antibiotics

10. The pharmaceutical composition according to claim 9, wherein said antibiotic is selected from tetracycline, amoxicillin and metronidazole.

11. The pharmaceutical composition according to claim 9 or 10 wherein said pharmaceutically acceptable carrier is selected from glucose and lactose.

12. The pharmaceutical composition according to any one of claims 8 to 11 in the form of granules.

13. A method for preparing the pharmaceutical composition according to any one of claims 8 to 12 wherein said method comprises:
    (a) passing whey obtained from bovine colostrum at least once through an ultrafiltration membrane to produce a retentate, wherein said membrane is impermeable to proteins having molecular weights greater than 100k daltons; and
    (b) lyophilizing said retentate to produce said pharmaceutical composition.

14. The method according to claim 13, further comprising passing said whey through a clarifying filter having pore size no greater than 0.2 μm prior to step (a).

15. The method according to claim 13 or 14 wherein said whey is obtained from cows previously immunized with *H. pylori* and optionally adjuvant.

16. The method according to any one of claims 13 to 15, further comprising adding a pharmaceutically acceptable carrier.

17. A method for preparing the pharmaceutical composition according to any one of claims 8 to 12 , wherein said method comprises:
    (a) passing a solution containing anti-*H. pylori* polyclonal immunoglobulins through an affinity column to absorb said antibodies on said column, wherein said affinity column is packed with beads containing dead *H. pylori* cells as absorbent; and
    (b) eluting said antibodies from said column.

18. The method according to claim 17, wherein said solution is bovine colostrum or whey.

19. The method according to claim 18, wherein said bovine colostrum or whey is first passed at least once through an ultrafiltration membrane, wherein said membrane is impermeable to proteins having molecular weights greater than 100k daltons.

20. The method according to any one of claims 17 to 19, further comprising adding a pharmaceutically acceptable carrier.

EP 0 484 148 A1

**European Patent Office**

## PARTIAL EUROPEAN SEARCH REPORT

which under Rule 45 of the European Patent Convention shall be considered, for the purposes of subsequent proceedings, as the European search report

Application Number

EP 91 31 0049

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | THE JOURNAL OF INFECTIOUS DISEASES, vol. 162, no. 1, July 1990, pages 156-162, The University of Chicago, Chicago, US; M.F. TOSI et al.: "Opsonic activity of specific human IgG against Helicobacter pylori" * The whole article * | 1,3,4,8 -20 | A 61 K 39/40 C 07 K 3/26 // (A 61 K 39/40 A 61 K 31:65 ) (A 61 K 39/40 A 61 K 31:43 ) (A 61 K 39/40 A 61 K 31:41 ) |
| Y | FR-A-2 387 039 (SOCIETE DES PRODUITS NESTLE S.A.) * Claims * | 1,3,4,8 -20 | |
| A | EP-A-0 367 644 (INSTITUT PASTEUR) * Claims * | 1,3,4,8 -12 | |
| A | THE NEW ENGLAND JOURNAL OF MEDICINE, vol. 138, no. 19, 12th May 1988, pages 1240-1243, Boston, US; C.O. TACKET et al.: "Protection by milk immunoglobulin concentrate against oral challenge with enterotoxigenic Escherichia coli" * Abstract * -/- | 1,3,4,8 -20 | |

TECHNICAL FIELDS SEARCHED (Int. Cl.5)

A 61 K
C 07 K
C 12 P

### INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 1,3,4,8-20
Claims searched incompletely :
Claims not searched : 2,5-7 because of EPC Art. 52(4)
Reason for the limitation of the search:

Remark : Although claims 1,3 and 4 are directed to a method of treatment of the human or animal body, the search has been carried out and based on the alleged effects of the compound.

| Place of search | Date of completion of the search | Examiner |
|---|---|---|
| THE HAGUE | 11-02-1992 | NOOIJ F.J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

13

**European Patent Office**

**PARTIAL EUROPEAN SEARCH REPORT**

Application Number

EP 91 31 0049

## DOCUMENTS CONSIDERED TO BE RELEVANT

| Category | Citation of document with indication, where appropriate, of relevant passages | Relevant to claim |
|---|---|---|
| A | EP-A-0 391 416  (CHUGAI SEIYAKU KABUSHIKI KAISHA)<br>* Claims *<br>--- | 1,3,4,8 -20 |
| A | THE LANCET, 1st April 1989, pages 690-692, London, GB; G. ODERDA et al.: "Amoxycillin plus tinidazole for campylobacter pylori gastritis in children: Assessment by serum IgG antibody, pepsinogen I, and gastrin levels"<br>* The whole article *<br>----- | 3,4,9, 10 |

**CLASSIFICATION OF THE APPLICATION (Int. Cl.5)**

**TECHNICAL FIELDS SEARCHED (Int. Cl.5)**

EPO FORM 1503 03.82 (P0410)